# EUROPEAN PATENT SPECIFICATION

(11) **EP 4 082 656 B1**
(45) Date of publication and mention of the grant of the patent: **19.02.2025**
(21) Application number: 20905473.3
(22) Date of filing: 28.12.2020
(51) Int. Cl.: B01J 13/14, C11B 9/00

(54) **METHOD FOR PRODUCING MICROCAPSULE**
VERFAHREN ZUR HERSTELLUNG VON MIKROKAPSELN
PROCÉDÉ DE FABRICATION DE MICROCAPSULES

(30) Priority: 27.12.2019 JP 2019239905
(43) Date of publication of application: 02.11.2022
(73) Proprietor: Kao Corporation, Chuo-ku Tokyo 103-8210 (JP)
(72) Inventor: KOGA, Yoshito, Wakayama-shi, Wakayama 640-8580 (JP)
(74) Representative: Hoffmann Eitle
(86) International application number: PCT/JP2020/049259
(87) International publication number: WO 2021/132730

(56) References cited:
- JP-A- 2013 255 915
- JP-A- 2015 128 762
- JP-A- 2017 165 623
- US-A1- 2012 104 638
- US-A1- 2012 104 639
- US-A1- 2012 295 790
- US-A1- 2018 078 468
- US-A1- 2018 242 665

## Description

### Field of the Invention

The present invention relates to a method of producing a microcapsule.

### Background of the Invention

In a wide range of business fields such as cosmetics, pharmaceuticals, general household goods, and printing, various microcapsules encapsulating fragrances or bioactive agent substances have been developed and used. For example, as for shells constituting microcapsules, aminoplast resins such as a melamine resin, or a polyurea/urethane resin have been used. However, microcapsules are inevitably discharged into the environment, and have recently become one of factors of substances of concern called microplastics. Thus, it is required to develop microcapsules with high environmental friendliness in replacement of aminoplast resins.

Among them, silica microcapsules (hereinafter, also called "silica capsules") having shells whose constituent component is silica are attracting attention as a material that may be expected to be environmentally friendly.

Silica capsules are generally obtained by a method of forming silica on the surfaces of emulsified droplets through a sol-gel method. Since the silica capsule itself is a very fine particle, the shell of the silica capsule is also very thin and brittle. Thus, a part of the encapsulated component of the silica capsule is eluted to the external environment by collapse of the shell, dissolution into the shell, or diffusion through micropores present in the shell. Therefore, until now, various methods of producing silica capsules by using a sol-gel method have been examined.

For example, JP 2013-255915 A (PTL 1) describes a method of producing microcapsules having a core substance, such as an active ingredient for a sunscreen. The described method of producing the microcapsules includes a step of producing an oil drop-in-water type emulsion by emulsifying an oil phase composed of a water-insoluble precursor and a core substance, in an aqueous phase composed of an aqueous solution having a predetermined pH, in which the aqueous phase used for forming a microcapsule oil drop-in-water type emulsion contains a cationic surfactant.

For the purpose of providing a method of producing microcapsules capable of retaining an organic compound as an active ingredient, such as a fragrance, for a long time, JP 2015-128762 A (PTL 2) describes a method of producing a microcapsule having a core made of an organic compound such as a fragrance, a first shell that encloses the core, and a second shell that encloses the first shell, in which a first sol-gel reaction step is performed in a state where the organic phase containing an organic compound and tetraalkoxysilane is emulsified in an aqueous phase containing a surfactant, and subsequently a second sol-gel reaction step is performed by adding tetraalkoxysilane, and maintaining the pH lower than that in the first sol-gel reaction step so as to obtain silica shells with a high shell density.

US 2012/0295790 Al relates to a microcapsule exhibiting desirable sustained-release properties, which microcapsule comprises a core material comprising an active ingredient encapsulated within a silica shell, characterized in that the outer surface of such silica shell has a layer of a metal selected from Group 2a, Group 8, Group 2b or Group 3a of the Periodic Table bound thereto.

### Summary of the Invention

The present invention relates to a method of producing a microcapsule that includes a shell containing silica as a constituent component and a core containing at least one organic compound inside the shell. The production method of the microcapsule includes:
step 1-1: subjecting an emulsion obtained by emulsifying an aqueous phase component comprising a cationic surfactant, and an oil phase component comprising at least one organic compound and a silica source to a sol-gel reaction under an acidic condition to form a silica microcapsule (1) that has a core and a first shell whose constituent component is silica, and to obtain a water dispersion of the silica microcapsule (1) containing an organic compound, and
step 2-1: adding an anionic surfactant to the water dispersion obtained in the step 1-1, and then performing a sol-gel reaction in the presence of a silica source to form a microcapsule having a shell that encloses the silica microcapsule (1).
Alternatively, the production method of the microcapsule includes:
Step 1-1: subjecting an emulsion obtained by emulsifying an aqueous phase component comprising a cationic surfactant, and an oil phase component comprising at least one organic compound and a silica source to a sol-gel reaction under an acidic condition to form a silica microcapsule (1) that has a core and a first shell whose constituent component is silica, and to obtain a water dispersion comprising the silica microcapsule (1), and
Step 2-2: adding an anionic surfactant and a silica source to the water dispersion obtained in the step 1-1, and then performing a sol-gel reaction of the silica source, to form a microcapsule having a second shell that encloses the silica microcapsule (1).

### Detailed Description of the Invention

In the techniques of PTLs 1 and 2, it was founded that probably because the denseness or strength of shells is insufficient, a functional oil agent such as a fragrance, especially, a fragrance having a low interfacial tension, cannot be sufficiently stably encapsulated, and the encapsulation rate is reduced by operations such as filtration after silica capsules are obtained.

The present invention relates to a method of producing a microcapsule capable of stably encapsulating an organic compound as an active component, such as a fragrance, at a high encapsulation rate.

Regarding the method of producing a microcapsule in which a shell of the microcapsule is formed through a sol-gel reaction step, the present inventor has focused on the fact that a silica source such as colloidal silica present in the system affects the denseness and sturdiness of the shells, and has found that due to the presence of an anionic surfactant during a sol-gel reaction of the silica source, a microcapsule having dense and sturdy shells and encapsulating an organic compound as a core component at a high encapsulation rate is obtained.

That is, the present invention relates to the following [1] to [2].
[1] A method of producing a microcapsule that includes a shell containing silica as a constituent component and a core containing at least one organic compound inside the shell. The production method of the microcapsule includes:
   step 1-1: subjecting an emulsion obtained by emulsifying an aqueous phase component comprising a cationic surfactant, and an oil phase component comprising at least one organic compound and a silica source to a sol-gel reaction under an acidic condition to form a silica microcapsule (1) that has a core and a first shell whose constituent component is silica, and to obtain a water dispersion of the silica microcapsule (1) containing an organic compound, and
   step 2-1: adding an anionic surfactant to the water dispersion obtained in the step 1-1, and then performing a sol-gel reaction in the presence of a silica source to form a microcapsule having a shell that encloses the silica microcapsule (1).
[2] A method of producing a microcapsule that includes a shell containing silica as a constituent component and a core containing at least one organic compound inside the shell. The production method of the microcapsule includes:
   step 1-1: subjecting an emulsion obtained by emulsifying an aqueous phase component comprising a cationic surfactant, and an oil phase component comprising at least one organic compound and a silica source to a sol-gel reaction under an acidic condition to form a silica microcapsule (1) that has a core and a first shell whose constituent component is silica, and to obtain a water dispersion of thesilica microcapsule (1) containing an organic compound, and
   step 2-2: adding an anionic surfactant and a silica source to the water dispersion obtained in the step 1-1, and then performing a sol-gel reaction of the silica source, to form a microcapsule having a second shell that encloses the silica microcapsule (1).

According to the present invention, it is possible to provide a method of producing a microcapsule capable of stably encapsulating an organic compound as an active component, such as a fragrance, at a high encapsulation rate.

### [Method of producing microcapsule]

The production method of a microcapsule (silica capsule) of the present invention is a method of producing a microcapsule that includes a shell containing silica as a constituent component and a core containing at least one organic compound inside the shell, and includes
Step 1-1: subjecting an emulsion obtained by emulsifying an aqueous phase component comprising a cationic surfactant, and an oil phase component comprising at least one organic compound and a silica source to a sol-gel reaction under an acidic condition to form a silica microcapsule (1) that has a core and a first shell whose constituent component is silica, and to obtain a water dispersion of the silica microcapsule (1) containing an organic compound, and
Step 2-1: adding an anionic surfactant to the water dispersion obtained in the step 1-1, and then performing a sol-gel reaction in the presence of a silica source to form a microcapsule having a shell that encloses the silica microcapsule (1). Alternatively, the production method of the microcapsule includes:
   Step 1-1: subjecting an emulsion obtained by emulsifying an aqueous phase component comprising a cationic surfactant, and an oil phase component comprising at least one organic compound and a silica source to a sol-gel reaction under an acidic condition to form a silica microcapsule (1) that has a core and a first shell whose constituent component is silica, and to obtain a water dispersion comprising the silica microcapsule (1), and
   Step 2-2: adding an anionic surfactant and a silica source to the water dispersion obtained in the step 1-1, and then performing a sol-gel reaction of the silica source, to form a microcapsule having a second shell that encloses the silica microcapsule (1).

The microcapsule related to the present invention is obtained through two sol-gel reaction steps from the viewpoint of improving the encapsulation rate of the organic compound. That is, the production method of the microcapsule of the present invention is a production method of a microcapsule (silica capsule) that includes a shell containing silica as a constituent component and a core containing at least one organic compound inside the shell, and relates to a microcapsule production method including the following step 1-1, and the step 2-1 or the step 2-2.

This includes:
Step 1-1: subjecting an emulsion obtained by emulsifying an aqueous phase component containing a cationic surfactant, and an oil phase component containing at least one organic compound and a silica source, to a sol-gel reaction under an acidic condition, to form a silica microcapsule (1) (silica capsule (1)) that has a core, and a first shell whose constituent component is silica, and to obtain a water dispersion containing the silica capsule (1),
Step 2-1: adding an anionic surfactant to the water dispersion obtained in the step 1-1, and then performing a sol-gel reaction of the silica source, to form a microcapsule having a second shell that encloses the silica microcapsule (1), or
Step 2-2: adding an anionic surfactant and a silica source to the water dispersion obtained in the step 1-1, and then, performing a sol-gel reaction of the silica source, to form a microcapsule having a second shell that encloses the silica microcapsule (1).

In the present invention, the "silica source" means a substance capable of forming a shell of a silica capsule, and examples thereof include a substance that produces a silanol compound through hydrolysis, such as tetraalkoxysilane. Further, in the present invention, the "silica source" includes silica sol and colloidal silica produced from a substance capable of forming a shell.

The silica source present in the silica capsule (1)-containing water dispersion may be confirmed with the naked eye, or the microscope in some cases, but may also be confirmed by the turbidity of an aqueous phase and measuring Si through an inductively coupled plasma (ICP) analysis when the aqueous phase and a capsule phase are separated by using ion chromatography or performing centrifugation.

In the present invention, the "sol-gel reaction" means a reaction in which the silica source forms silica that is a constituent component of the shell through a sol and gel state by hydrolysis and polycondensation reactions. Specifically, for example, in the reaction, tetraalkoxysilane is hydrolyzed, a silanol compound produces a siloxane oligomer through a dehydration condensation reaction and a dealcoholization condensation reaction, and further a dehydration condensation reaction proceeds so as to form silica.

According to the production method of the present invention, it is possible to stably encapsulate an organic compound as an active component, such as a fragrance, at a high encapsulation rate. The reason is not clear, but is thought to be as follows.

In the method of producing a silica capsule, in which a shell of the silica capsule is formed through a sol-gel reaction step, an anionic surfactant is added to a silica capsule-containing water dispersion in which a silica source such as colloidal silica is present in the system, and then it is thought that since the anionic surfactant is present during a sol-gel reaction of the silica source, the anionic surfactant is preferentially adsorbed onto the colloidal silica that exists in the system without forming shells. As a result, it is thought that the colloidal silica is further negatively charged, and thus is easily adsorbed on cationic silica capsule shells, and then due to an increase of the amount of silica layered on the shell surfaces or an improvement of denseness of the shells, even after undergoing operations such as filtration, a silica capsule having dense and sturdy shells and retaining an organic compound as a core component at a high encapsulation rate is obtained.

Further, in the method of producing a silica capsule, in which a shell of the silica capsule is formed through two sol-gel reaction steps, a cationic surfactant is used as an emulsifier of an emulsion to be provided to a first sol-gel reaction step, so that the formed first shells are placed in a state where their surfaces are positively charged by the cationic surfactant. Then, it is thought that since an anionic surfactant is present during a second sol-gel reaction step, the anionic surfactant is preferentially adsorbed onto colloidal silica that exists in the system without forming shells. As a result, it is thought that the colloidal silica is further negatively charged, and thus is easily adsorbed on the positively charged first shells, and then due to an increase of the amount of silica layered on the surfaces of the first shells or an improvement of denseness of the shells, even after undergoing operations such as filtration, a silica capsule having dense and sturdy shells and retaining an organic compound as a core component at a high encapsulation rate is obtained.

Then, it is thought that the above-described effect of obtaining a silica capsule having dense and sturdy shells may be probably certainly an effect caused by the improvement of denseness of the shells together with the increase of the amount of silica.

### (Step 1-1: obtaining water dispersion of silica capsule (1))

The step 1-1 is a step of subjecting an emulsion obtained by emulsifying an aqueous phase component comprising a cationic surfactant, and an oil phase component comprising at least one organic compound and a silica source to a sol-gel reaction under an acidic condition to form a silica microcapsule (1) that has a core and a first shell whose constituent component is silica, and to obtain a water dispersion of the silica microcapsule (1) (silica capsule (1)) containing an organic compound.

The water dispersion obtained in the step 1-1 contains the silica capsule (1) having a shell that contains silica as a constituent component, and a core that contains at least one organic compound inside the shell.

### <Silica source>

The silica source preferably contains tetraalkoxysilane as a main component from the viewpoint of obtaining a silica capsule having dense and sturdy shells, and is more preferably tetraalkoxysilane having an alkoxy group having 1 or more and 4 or less carbon atoms, further preferably one or more selected from the group consisting of tetramethoxysilane, tetraethoxysilane, and tetraisopropoxysilane, still further preferably one or more selected from the group consisting of tetramethoxysilane and tetraethoxysilane, still further preferably tetraethoxysilane.

When the silica source contains tetraalkoxysilane, trialkoxysilane such as triethoxysilane, or trimethoxysilane may be contained, but the content of tetraalkoxysilane in the silica source is preferably 80% by mass or more, more preferably 85% by mass or more, further preferably 90% by mass or more, and is preferably 100% by mass or less.

The amount of the silica source to be added in order to form the silica microcapsule (1) in the step 1-1 is preferably 10% by mass or more, more preferably 15% by mass or more, further preferably 20% by mass or more based on the amount of the organic compound in the step 1-1 from the viewpoint of forming shells surrounding peripheries of oil phase emulsified droplets containing the organic compound, and is preferably 100% by mass or less, more preferably 70% by mass or less, further preferably 50% by mass or less, still further preferably 40% by mass or less, still further preferably 30% by mass or less from the viewpoint of suppressing the silica source from remaining inside oil phase droplets, and efficiently promoting the conversion into shells.

### [Step 1-1]

The step 1-1 is the following step 1-1 from the viewpoint of improving the encapsulation rate of the organic compound.

Step 1-1: subjecting an emulsion obtained by emulsifying an aqueous phase component containing a cationic surfactant, and an oil phase component containing at least one organic compound and a silica source to a sol-gel reaction under an acidic condition, to form a silica capsule (1) that has a core, and a first shell whose constituent component is silica, and to obtain a water dispersion of the silica capsule (1).

### <Cationic surfactant>

Examples of the cationic surfactant used for producing the silica capsule (1)-water dispersion include alkylamine salts, and alkyl quaternary ammonium salts. The number of carbon atoms in the alkyl group of the alkylamine salt and the alkyl quaternary ammonium salt is preferably 10 or more, more preferably 12 or more, further preferably 14 or more, and is preferably 22 or less, more preferably 20 or less, further preferably 18 or less.

Examples of the alkylamine salt include alkylamine acetates such as laurylamine acetate, and stearylamine acetate.

Examples of the alkyl quaternary ammonium salt include an alkyltrimethyl ammonium salt, a dialkyldialkyl ammonium salt, and an alkylbenzyldimethyl ammonium salt.

Examples of the alkyltrimethyl ammonium salt include alkyltrimethyl ammonium chlorides such as lauryltrimethyl ammonium chloride, cetyltrimethyl ammonium chloride, and stearyltrimethyl ammonium chloride; and alkyltrimethyl ammonium bromides such as lauryltrimethyl ammonium bromide, cetyltrimethyl ammonium bromide, and stearyltrimethyl ammonium bromide.

Examples of the dialkyldimethyl ammonium salt include dialkyldimethyl ammonium chlorides such as distearyldimethyl ammonium chloride; and dialkyldimethyl ammonium bromides such as distearyldimethyl ammonium bromide.

Examples of the alkylbenzyldimethyl ammonium salt include alkylbenzyldimethyl ammonium chloride, and alkylbenzyldimethyl ammonium bromide.

The cationic surfactant may be used alone or in combination of two or more thereof.

Among these, the cationic surfactant is preferably a quaternary ammonium salt, more preferably an alkyltrimethyl ammonium salt having an alkyl group having 10 or more and 22 or less carbon atoms, further preferably one or more selected from the group consisting of lauryltrimethyl ammonium chloride, stearyltrimethyl ammonium chloride, and cetyltrimethyl ammonium chloride, still further preferably cetyltrimethyl ammonium chloride.

The amount of the cationic surfactant used for producing the silica capsule (1)-water dispersion is preferably 0.3% by mass or more, more preferably 0.5% by mass or more, further preferably 0.7% by mass or more, still further preferably 1% by mass or more, still further preferably 1.5% by mass or more, and is preferably 5% by mass or less, more preferably 4% by mass or less, further preferably 3% by mass or less, still further preferably 2.5% by mass or less based on the amount of the organic compound in the step 1-1 from the viewpoint of obtaining a stable emulsion.

### <Organic compound>

The core of the silica capsule (1) related to the present invention contains at least one organic compound.

The organic compound is preferably one or more selected from the group consisting of fragrances, fragrance precursors, oil agents (for example, moisturizers), antioxidants, antibacterial agents, fertilizers, surface modifiers for fiber, skin, hair, etc., cold sense agents, dyes, pigments, silicone, solvents, and oil-soluble polymers, more preferably one or more selected from the group consisting of fragrances, fragrance precursors, oil agents, antioxidants, antibacterial agents, fertilizers, surface modifiers, and solvents, further preferably one or more selected from the group consisting of fragrances, fragrance precursors, oil agents, antioxidants, and solvents, still further preferably one or more selected from the group consisting of fragrances, fragrance precursors and oil agents, still further preferably one or more selected from the group consisting of fragrances, fragrance precursors and moisturizers, still further preferably one or more selected from the group consisting of fragrances and fragrance precursors.

As for the organic compound, one type may be used alone or two or more types may be used.

Examples of the fragrance precursor include a compound that releases a fragrance component by reacting with water, and a compound that releases a fragrance component by reacting with light.

Examples of the compound that releases a fragrance component by reacting with water include a silicic acid ester compound containing an alkoxy component derived from fragrance alcohol, a fatty acid ester compound containing an alkoxy component derived from fragrance alcohol, an acetal compound or a hemiacetal compound obtained through a reaction between a carbonyl component derived from fragrance aldehyde or fragrance ketone and an alcohol compound, a Schiff base compound obtained through a reaction between a carbonyl component derived from fragrance aldehyde or fragrance ketone and a primary amine compound, and a hemiaminal compound or a hydrazone compound obtained through a reaction between a carbonyl component derived from fragrance aldehyde or fragrance ketone and a hydrazine compound.

Examples of the compound that releases a fragrance component by reacting with light include a 2-nitrobenzylether compound containing an alkoxy component derived from fragrance alcohol, an α-keto ester compound containing a carbonyl component derived from fragrance aldehyde or fragrance ketone, and a coumaric acid ester compound containing an alkoxy component derived from fragrance alcohol. These fragrance precursors may be used, for example, as a polymer such as a product of reaction between some carboxy groups of polyacrylic acid and fragrance alcohol.

It is desirable that the organic compound has an appropriate hydrophobicity from the viewpoint of obtaining a stable emulsion. As for the index indicating the hydrophilicity or hydrophobicity of the organic compound, a cLogP value, which is a calculated value of a common logarithm "LogP" of a partition coefficient P (n-octanol/water) between n-octanol and water, may be used. The cLogP value is "LogP (cLogP)" calculated by the method described in A. Leo Comprehensive Medicinal Chemistry, Vol.4 C. Hansch, P. G. Sammens, J. B Taylor and C. A. Ramsden, Eds., P.295, Pergamon Press, 1990, and is a cLogP value calculated by a program CLOGP v4.01.

When the organic compound is composed of a plurality of constituent components, the cLogP value of the organic compound may be obtained by multiplying respective cLogP values of the constituent components by respective volume ratios of the constituent components, and summing up these.

The cLogP value of the organic compound is preferably 1 or more, more preferably 2 or more, further preferably 3 or more, and is preferably 30 or less, more preferably 20 or less, further preferably 10 or less.

When the cLogP value of the organic compound is 1 or more, in a sol-gel reaction using oil drops in water, which will be described below, the encapsulation rate of the organic compound within the obtained silica capsule (hereinafter, also called "encapsulation rate") is improved. Further, even in a case in which the organic compound is composed of a plurality of fragrance components, like a fragrance composition, similarly, when the cLogP value of the fragrance composition is 1 or more, the encapsulation rate of the fragrance composition within the silica capsule obtained through a sol-gel reaction may be improved.

Since in the production method of the present invention, the organic compound may be encapsulated at a high encapsulation rate, the organic compound may have a low interfacial tension.

It is thought that in a conventional method, in encapsulating an organic compound having a low oil-water interfacial tension, since the adsorption of silica sol onto a shell forming site is inhibited, a high-encapsulation-rate encapsulation was difficult. In contrast, it is thought that in the present invention, due to the addition of the anionic surfactant, since the adsorption of silica sol or colloidal silica as the silica source, onto a shell forming site, is promoted, a high-encapsulation-rate encapsulation may be achieved.

From this viewpoint, the oil-water interfacial tension of the organic compound is preferably 5 mN/m or more, more preferably 8 mN/m or more, further preferably 10 mN/m or more, and is preferably 35 mN/m or less, more preferably 30 mN/m or less, further preferably 25 mN/m or less, still further preferably 20 mN/m or less.

The oil-water interfacial tension of the organic compound may be measured by the method described in Examples.

The amount of the oil phase component is preferably 5% by mass or more with respect to the total amount of the emulsion obtained in the step 1-1, more preferably 10% by mass or more, further preferably 15% by mass or more, still further preferably 20% by mass or more from the viewpoint of production efficiency, and is preferably 50% by mass or less, more preferably 45% by mass or less, further preferably 40% by mass or less, from the viewpoint of obtaining a stable emulsion.

Further, the step 1-1 preferably includes the following steps 1-1-1 to 1-1-3.

Step 1-1-1: preparing an aqueous phase component containing a cationic surfactant.

Step 1-1-2: mixing at least one organic compound to be encapsulated into a silica capsule with a silica source to prepare an oil phase component.

Step 1-1-3: mixing and emulsifying the aqueous phase component obtained in the step 1-1-1 and the oil phase component obtained in the step 1-1-2 to obtain an emulsion.

Although a stirring unit used for mixing and emulsifying the aqueous phase component and the oil phase component is not particularly limited, a homogenizer having a strong shear force, a high-pressure disperser, an ultrasonic disperser, etc. may be used. Further, a homomixer, "DISPER" (product name, manufactured by Primix Corporation), "CLEARMIX" (product name, manufactured by M Technique Co. Ltd.), "CAVITRON" (product name, manufactured by Pacific Machinery & Engineering Co. Ltd.), etc. may also be used.

The median diameter D₅₀ of emulsified droplets of the emulsion in the step 1-1 is preferably 0.1 µm or more, more preferably 0.2 µm or more, further preferably 0.3 µm or more from the viewpoint of reducing the specific surface area with respect to the environment outside the silica capsule and increasing the encapsulation rate of the organic compound, and is preferably 50 µm or less, more preferably 30 µm or less, further preferably 10 µm or less, still further preferably 5 µm or less, still further preferably 3 µm or less from the viewpoint of the physical strength of the silica capsule.

The median diameter D₅₀ of the emulsified droplets may be measured by the method described in Examples.

The step 1-1 is a step of subjecting the above-described emulsion to a sol-gel reaction under an acidic condition, to form a silica capsule (1) that has a core, and a first shell whose constituent component is silica, and to obtain a water dispersion containing the silica capsule (1).

The initial pH of the sol-gel reaction in the step 1-1 is preferably 3.0 or more, more preferably 3.3 or more, further preferably 3.5 or more from the viewpoint of maintaining the balance between a hydrolysis reaction and a condensation reaction of tetraalkoxysilane, and the viewpoint of suppressing the formation of highly hydrophilic sol, and promoting the progress of encapsulation, and is preferably 4.5 or less, more preferably 4.3 or less, further preferably 4.1 or less from the viewpoint of suppressing co-occurrence of formation of a shell and aggregation of emulsified droplets and obtaining the silica capsule having a dense shell.

Any acidic or alkaline pH adjuster may be used in order to perform adjustment to a desired initial pH according to the intensity of acidity or alkalinity of the oil phase component containing the organic compound.

The pH of the emulsion may also be a desired value or less. In such a case, it is desirable that adjustment is performed by using an alkaline pH adjuster to be described below.

That is, the step 1-1 may preferably further include the following step 1-1-4.

Step 1-1-4: adjusting the pH of the obtained emulsion by using a pH adjuster.

Examples of the acidic pH adjuster include inorganic acids such as hydrochloric acid, nitric acid, and sulfuric acid, organic acids such as acetic acid, and citric acid, and solutions obtained by adding a cation exchange resin to water or ethanol. Hydrochloric acid, sulfuric acid, nitric acid, and citric acid are preferred.

Examples of the alkaline pH adjuster include sodium hydroxide, sodium hydrogen carbonate, potassium hydroxide, ammonium hydroxide, diethanolamine, triethanolamine, and trishydroxy methylaminomethane. Sodium hydroxide, and ammonium hydroxide are preferred.

As for the reaction temperature of the sol-gel reaction in the step 1-1, any value may be selected as long as it is equal to or greater than a melting point of water contained as the dispersion medium and is equal to or smaller than a boiling point. However, it is preferably adjusted to preferably 5°C or more, more preferably 10 °C or more, further preferably 15 °C or more, still further preferably 20 °C or more, and to preferably 60 °C or less, more preferably 50°C or less, further preferably 40 °C or less in order to control the balance between a hydrolysis reaction and a condensation reaction in the sol-gel reaction, and to form a dense shell.

### (Step 2-1 and Step 2-2: anionic surfactant addition and sol-gel reaction)

The step 2-1 and the step 2-2 are steps of adding an anionic surfactant to the water dispersion obtained in the step 1-1, and then performing a sol-gel reaction of a silica source, to form a silica capsule having a shell that encloses the silica capsule (1).

### <Anionic surfactant>

In the present invention, the anionic surfactant has an anionic group as a hydrophilic group and a lipophilic group, in the molecule from the viewpoint of increasing the encapsulation rate of the organic compound. Examples of the anionic group include groups that become acidic due to dissociation and release of hydrogen ions, such as a sulfate ester group (-OSOsM), a sulfonic acid group (-SOsM), a carboxy group (-COOM), and a phosphoric acid group (-OPO₃M₂), or their dissociated ionic forms (-OSO₃⁻, -SO₃⁻, -COO⁻, -OPO₃²⁻, -OPO₃⁻M). In the above chemical formula, M represents a counterion of the anionic group.

Examples of the anionic surfactant include sulfate ester salts such as a polyoxyethylene alkylether sulfate ester salt, a polyoxyethylene polyoxypropylene alkylether sulfate ester salt, and an alkyl sulfate ester salt; sulfonates such as alkylbenzene sulfonate; low molecular dispersants having anionic groups, such as alkenyl succinate, and carboxylate such as a fatty acid salt having 8 or more and 22 or less carbon atoms; and high molecular dispersants having anionic groups, such as a salt of aromatic sulfonic acid formalin condensate such as a sodium salt of β-naphthalene sulfonic acid formalin condensate, an acrylic acid homopolymer, an acrylic acid copolymer, a maleic acid homopolymer, and a maleic acid copolymer.

As for the anionic dispersant, one type may be used alone or two or more types may be used.

Examples of the counter ion of the anionic group of the anionic surfactant include alkali metal ions such as a sodium ion and a potassium ion; alkaline earth metal ions such as a calcium ion, and a magnesium ion; an ammonium ion; and alkanol ammonium having 1 to 3 alkanol groups having 2 or 3 carbon atoms (for example, monoethanol ammonium, diethanol ammonium, triethanol ammonium, and triisopropanol ammonium).

Among them, the anionic surfactant is preferably one or more selected from the group consisting of a low molecular dispersant having an anionic group, and a high molecular dispersant having an anionic group from the viewpoint of increasing the encapsulation rate of the organic compound, more preferably a low molecular dispersant having an anionic group, further preferably a sulfate ester salt, further preferably one or more selected from the group consisting of a polyoxyethylene alkylether sulfate ester salt, a polyoxyethylene polyoxypropylene alkylether sulfate ester salt, and an alkyl sulfate ester salt, still further preferably one or more selected from the group consisting of a polyoxyethylene alkylether sulfate ester salt, and an alkyl sulfate ester salt, still further preferably a polyoxyethylene alkylether sulfate ester salt.

In the step 2-1 and the step 2-2 to be described in detail below, a nonionic dispersant may coexist in a range where the effect of the present invention is not inhibited.

Examples of the nonionic dispersant include nonionic surfactants such as polyoxyethylene alkylether having 8 or more and 22 or less carbon atoms, sorbitol fatty acid ester, sorbitan fatty acid ester, glycerin fatty acid ester, pentaerythritol fatty acid ester, polyoxyethylene fatty acid ester, an alkylphenol ethylene oxide adduct, a higher alkylamine ethylene oxide adduct, and a polypropylene glycol ethylene oxide adduct.

In the step 2-1 and the step 2-2, the anionic surfactant is preferably added within a range where the surface of the silica capsule (1) is not negatively charged from the viewpoint of promoting layering of silica on the shell surface, improving the denseness of the shell, and increasing the encapsulation rate of the organic compound. From this viewpoint, the amount of the anionic surfactant to be added in the step 2-1 and the step 2-2 is preferably 0.1% by mass or more, more preferably 0.2% by mass or more, further preferably 0.3% by mass or more, and is preferably 3% by mass or less, more preferably 2% by mass or less, further preferably 1% by mass or less, still further preferably 0.5% by mass or less based on the amount of the silica capsule (1)-containing water dispersion.

### [Step 2-1 and Step 2-2]

Step 2-1: adding an anionic surfactant to the water dispersion obtained in the step 1-1, and then performing a sol-gel reaction, to form a microcapsule having a second shell that encloses the silica microcapsule (1).

Step 2-2: adding an anionic surfactant and a silica source to the water dispersion obtained in the step 1-1, and then performing a sol-gel reaction, to form a microcapsule having a second shell that encloses the silica microcapsule (1).

The amount of the anionic surfactant to be added in the step 2-1 or the step 2-2 is preferably 0.1% by mass or more, more preferably 0.2% by mass or more, further preferably 0.3% by mass or more, and is preferably 3% by mass or less, more preferably 2% by mass or less, further preferably 1% by mass or less, still further preferably 0.5% by mass or less based on the amount of the silica capsule (1)-containing water dispersion.

### <Silica source>

The silica source to be added in the step 2-2 preferably contains tetraalkoxysilane as a main component, and is more preferably tetraalkoxysilane having an alkoxy group having 1 or more and 4 or less carbon atoms from the viewpoint of promoting a sol-gel reaction, further preferably one or more selected from the group consisting of tetramethoxysilane, tetraethoxysilane, and tetraisopropoxysilane, still further preferably one or more selected from the group consisting of tetramethoxysilane and tetraethoxysilane, still further preferably tetraethoxysilane.

The silica source to be used in the step 2-2 may contain triethoxysilane or trimethoxysilane, but the content of tetraalkoxysilane in the silica source is preferably 80% by mass or more, more preferably 85% by mass or more, further preferably 90% by mass or more, and is preferably 100% by mass or less.

The amount of the silica source, preferably tetraalkoxysilane, to be added in the step 2-2 is, based on the amount of the organic compound in the step 1-1, preferably 5% by mass or more, more preferably 10% by mass or more, further preferably 13% by mass or more, from the viewpoint of forming the second shell that encloses the silica capsule (1), and is preferably 100% by mass or less, more preferably 70% by mass or less, further preferably 50% by mass or less, still further preferably 30% by mass or less, still further preferably 20% by mass or less from the viewpoint of suppressing formation of silica sol dispersed in the aqueous phase, improving the denseness and the physical strength of the shell, and improving the encapsulation property of the organic compound, and the viewpoint of improving the dispersion stability of the silica capsule.

The amount of the silica source, preferably tetraalkoxysilane, to be added in the step 2-2 is, based on the amount of the water dispersion obtained in the step 1-1, preferably 1% by mass or more, more preferably 1.5% by mass or more, further preferably 2% by mass or more, still further preferably 2.8% by mass or more from the viewpoint of forming the second shell that encloses the first shell, and is preferably 10% by mass or less, more preferably 7% by mass or less, further preferably 5% by mass or less from the viewpoint of suppressing formation of silica sol dispersed in the aqueous phase, improving the denseness and the physical strength of the shell, and improving the encapsulation property of the organic compound, and the viewpoint of improving the dispersion stability of the silica capsule.

The amount of the silica source, preferably tetraalkoxysilane, to be added in the step 2-2 is, based on the amount of the water dispersion obtained by adding the anionic surfactant to the water dispersion obtained in the step 1-1, preferably 1% by mass or more, more preferably 1.5% by mass or more, further preferably 2% by mass or more, still further preferably 2.8% by mass or more from the viewpoint of forming the second shell that encloses the first shell, and is preferably 10% by mass or less, more preferably 7% by mass or less, further preferably 5% by mass or less from the viewpoint of suppressing formation of silica sol dispersed in the aqueous phase, improving the denseness and the physical strength of the shell, and improving the encapsulation property of the organic compound, and the viewpoint of improving the dispersion stability of the silica capsule.

In the step 2-2, the entire amount of the silica source, preferably tetraalkoxysilane, to be added to the silica capsule (1)-water dispersion obtained in the step 1-1 may be added at once, or intermittent addition by division or continuous addition may be carried out.

The reaction temperature of the sol-gel reaction in the step 2-1 or the step 2-2 may be arbitrarily selected as long as it is equal to or greater than a melting point of water contained as a dispersion medium, and is equal to or smaller than a boiling point. However, it is preferably 5°C or more, more preferably 10°C or more, further preferably 15°C or more, still further preferably 20°C or more, and is preferably 60°C or less, more preferably 50°C or less, further preferably 40°C or less from the viewpoint of controlling the balance between a hydrolysis reaction and a condensation reaction in the sol-gel reaction, and forming a dense shell.

The sol-gel reaction of the step 1-1 and the sol-gel reaction in the step 2-1 or the step 2-2 may be carried out at different reaction temperatures.

### (Microcapsule)

The microcapsule (silica capsule) related to the present invention is obtained as a water dispersion dispersed in water. This may be used as the water dispersion, as it is, depending on purposes of the silica capsule, but, the silica capsule may also be separated from the water dispersion and then used depending on purposes. As for the separation method, a filtration method, a centrifugation method, etc. may be adopted.

The silica capsule related to the present invention has a core made of at least one organic compound, a first shell that encloses the core, and a second shell that encloses the first shell.

The first shell of the silica capsule related to the present invention encloses the core, contains silica as a constituent component, and preferably has an average thickness of 5 nm or more and 20 nm or less, and the second shell encloses the first shell, contains silica as a constituent component, and preferably has an average thickness of 10 nm or more and 100 nm or less.

The average thicknesses of the first shell and the second shell of the silica capsule related to the present invention may be measured by transmission electron microscope (TEM) observation. Specifically, under the observation of a transmission electron microscope, the thicknesses of the first shells and the second shells are actually measured on the photograph. This operation is performed by changing the field of view. From the obtained data, distributions of average thicknesses of the first shells and the second shells are obtained. The reference of magnification of the transmission electron microscope is 10,000 times or more and 100,000 times or less, but is appropriately adjusted according to the sizes of the silica capsule. Here, as for the transmission electron microscope (TEM), for example, "JEM-2100" (product name, manufactured by JEOL Ltd.) may be used.

The median diameter D₅₀ of the silica capsule related to the present invention is preferably 0.1 µm or more, more preferably 0.5 µm or more, further preferably 1 µm or more, from the viewpoint of improving the encapsulation rate of the organic compound, and the viewpoint of improving the dispersion stability of the silica capsule, and is preferably 50 µm or less, more preferably 30 µm or less, further preferably 10 µm or less from the viewpoint of improving the physical strength of the silica capsule, and improving the encapsulation rate of the organic compound.

The median diameter D₅₀ of the silica capsule may be measured by the method described in Examples.

The silica capsule related to the present invention may be used for various purposes, and may be highly suitably used for, for example, various purposes, such as cosmetics, e.g., milky lotion, cosmetic liquid, cosmetic water, beauty serum, cream, gel formulation, hair treatment, and quasi-drugs, fiber treatment agents, e.g., a detergent, a softener, and an anti-wrinkle spray, sanitary products, e.g., paper diapers, and air fresheners.

The silica capsule related to the present invention may be used by being blended with a composition such as a detergent composition, a fiber treatment agent composition, a cosmetic composition, an air freshener composition, and a deodorant composition. As the composition, a detergent composition such as a powder detergent composition or a liquid detergent composition, and a fiber treatment agent composition such as a softener composition are preferred. Among these, a fiber treatment agent composition is more preferred, and a softener composition is further preferred.

### [Example]

Various measurements used in Examples and Comparative Examples were performed by the following methods.

### [Median diameter D₅₀]

The median diameter D₅₀ of emulsified droplets and the median diameter D₅₀ of silica capsules were measured by using a laser diffraction/scattering particle diameter distribution measuring device "LA-960" (product name, manufactured by HORIBA, Ltd.). The measurement was performed using a flowcell, the medium was water, and the refractive index of a dispersoid was set as 1.45-0i. An emulsion or a water dispersion containing silica capsules was added into the flowcell, and the measurement was carried out at a concentration at which a transmittance of around 90% was exhibited so as to obtain the median diameter D₅₀ on a volume basis.

### [Oil-water interfacial tension]

The oil-water interfacial tension of an organic compound encapsulated in the silica capsule was measured by a hanging drop method (pendant drop method). In a constant temperature room of 25 °C, a contact angle meter "DropMaster series DM-501" (manufactured by Kyowa Interface Science Co. Ltd) was used. The analysis was performed through the Young-Laplace method by using software "FAMAS" (manufactured by Kyowa Interface Science Co. Ltd).

### <Model fragrance A>

As for the organic compound to be encapsulated in the silica capsule, a model fragrance A having a composition noted in Table 1 (volume average cLogP value: 3.7, specific gravity: 0.96, oil-water interfacial tension: 11.6 mN/m) was used. The volume average cLogP value of the model fragrance was calculated by multiplying the cLogP values of all the fragrance components contained in the model fragrance by volume ratios thereof in the model fragrance, respectively, and summing up these.

**Table 1 : Model fragrance A**

| Fragrance component name | Content (% by mass) | cLogP |
|---|---|---|
| Linalool | 4 | 3.3 |
| Rose oxide | 4 | 3.6 |
| Menthone | 4 | 2.9 |
| Citronellol | 42 | 3.6 |
| Geraniol | 21 | 3.5 |
| Linalyl acetate | 17 | 4.4 |
| Others | 8 | |

### Example 1

### (Step 1-1)

0.50 g of QUARTAMIN 60W (product name, manufactured by Kao corporation; cetyltrimethylammonium chloride (CTAC), active component 30% by mass) was diluted with 74.50 g of ion-exchanged water to prepare an aqueous phase component. To this aqueous phase component, an oil phase component, which was prepared by mixing 20 g of a model fragrance A with 5 g of tetraethoxysilane (hereinafter, also referred to as "TEOS"), was added. The mixed solution was emulsified by using a homomixer (manufactured by HsiangTai, model: HM-310) set at a rotation speed of 7,000 rpm and then further emulsified after the rotation speed of the homomixer was changed to 8,500 rpm. Then, an emulsion was obtained. Here, the median diameter D₅₀ of emulsified droplets was 1.1 µm.

Next, after the pH of the obtained emulsion was adjusted to 3.8 by using 0.09 g of 0.2 N hydrochloric acid, the emulsion was transferred to a separable flask equipped with a stirring blade. Then, while the liquid temperature was maintained at 30°C, stirring was performed at 200 rpm for 24 h to obtain a water dispersion containing a silica capsule (1-1) that has a core made of the model fragrance A and a first shell made of silica.

As a result of ICP (inductively coupled plasma) analysis of the obtained water dispersion, Si was detected, and thus it was confirmed that a silica source was present in the water dispersion.

### (Step 2-2)

To 13 g taken out from the total amount of 100.09 g of the water dispersion obtained in the step 1-1, 0.17 g of EMAL 125A (product name, manufactured by Kao corporation; polyoxyethylene alkyl ether sulfate ester salt, active component 25% by mass) was added, and through uniform mixing, the water dispersion of silica capsules was obtained. Next, to the water dispersion, 0.43 g of TEOS was added. Then, the mixture was stirred for 24 h while the liquid temperature was maintained at 30 °C, and then was cooled to a room temperature so as to form a second shell that encloses the silica capsule. Then, a water dispersion containing a silica capsule (A1) in which the model fragrance A was encapsulated in amorphous silica was obtained. The median diameter D₅₀ of the silica capsule (A1) was 2.9 µm.

### Example 2

### (Step 1-1)

2.50 g of QUARTAMIN 60W (product name, manufactured by Kao corporation; CTAC, active component 30% by mass) was diluted with 148.75 g of ion-exchanged water to prepare an aqueous phase component. To this aqueous phase component, an oil phase component, which was prepared by mixing 40 g of a model fragrance A with 10 g of TEOS, was added. The mixed solution was emulsified by using the homomixer set at a rotation speed of 7,000 rpm and then further emulsified after the rotation speed of the homomixer was changed to 8,500 rpm. Then, an emulsion was obtained. Here, the median diameter D₅₀ of emulsified droplets was 1.0 µm.

Next, after the pH of the obtained emulsion was adjusted to 3.8 by using 0.15 g of 0.2 N hydrochloric acid, the emulsion was transferred to a separable flask equipped with a stirring blade. Then, while the liquid temperature was maintained at 30°C, stirring was performed at 200 rpm for 24 h to obtain a water dispersion containing a silica capsule (1-2) that has a core made of the model fragrance A and a first shell made of silica.

As a result of ICP (inductively coupled plasma) analysis of the obtained water dispersion, Si was detected, and thus it was confirmed that a silica source was present in the water dispersion.

### (Step 2-2)

To 13 g taken out from the total amount of 201.4 g of the water dispersion obtained in the step 1-1, 0.16 g of EMAL 125A (product name, manufactured by Kao corporation; polyoxyethylene alkylether sulfate ester salt, active component 25% by mass) was added to obtain the water dispersion of silica capsules. Next, to the water dispersion, 0.40 g of TEOS was added. Then, the mixture was stirred for 24 h while the liquid temperature was maintained at 30°C, and then was cooled to a room temperature so as to form a second shell that encloses the silica capsule. Then, a water dispersion containing a silica capsule (A2) in which the model fragrance A was encapsulated in amorphous silica was obtained. The median diameter D₅₀ of the silica capsule (A2) was 2.9 µm.

### Example 3

### Step 1-1)

A water dispersion containing a silica capsule (1-3) was obtained through the same operation as in Example 2 (step 1-1).

As a result of ICP (inductively coupled plasma) analysis of the obtained water dispersion, Si was detected, and thus it was confirmed that a silica source was present in the water dispersion.

### (Step 2-2)

To 13 g taken out from the total amount of 201.4 g of the water dispersion obtained in the step 1-1, 0.20 g of LATEMUL E-108MB (product name, manufactured by Kao corporation; polyoxyethylene alkylether sulfate ester salt, active component 20% by mass) was added to obtain the water dispersion of silica capsules. Next, to the water dispersion, 0.40 g of TEOS was added. Then, the mixture was stirred for 24 h while the liquid temperature was maintained at 30°C, and then was cooled to a room temperature so as to form a second shell that encloses the silica capsule. Then, a water dispersion containing a silica capsule (A3) in which the model fragrance A was encapsulated in amorphous silica was obtained. The median diameter D₅₀ of the silica capsule (A3) was 8.0 µm.

### Example 4

### (Step 1-1)

A water dispersion containing a silica capsule (1-4) was obtained through the same operation as in Example 2 (step 1-1).

As a result of ICP (inductively coupled plasma) analysis of the obtained water dispersion, Si was detected, and thus it was confirmed that a silica source was present in the water dispersion.

### (Step 2-2)

To 13 g taken out from the total amount of 201.4 g of the water dispersion obtained in the step 1-1, 0.13 g of EMAL TD (product name, manufactured by Kao corporation; triethanolamine lauryl sulfate, active component 40% by mass) was added to obtain the water dispersion of silica capsules. Next, to the water dispersion, 0.40 g of TEOS was added. Then, the mixture was stirred for 24 h while the liquid temperature was maintained at 30°C, and then was cooled to a room temperature so as to form a second shell that encloses the silica capsule. Then, a water dispersion containing a silica capsule (A4) in which the model fragrance A was encapsulated in amorphous silica was obtained. The median diameter D₅₀ of the silica capsule (A4) was 3.8 µm.

### Example 5

### (Step 1-1)

A water dispersion containing a silica capsule (1-5) was obtained through the same operation as in Example 2 (step 1-1).

As a result of ICP (inductively coupled plasma) analysis of the obtained water dispersion, Si was detected, and thus it was confirmed that a silica source was present in the water dispersion.

### (Step 2-2)

To 13 g taken out from the total amount of 201.4g of the water dispersion obtained in the step 1-1, 0.13 g of EMAL 20T (product name, manufactured by Kao corporation; triethanolamine polyoxyethylene (3) lauryl ether sulfate, active component 40% by mass) was added to obtain the water dispersion of silica capsules. Next, to the water dispersion, 0.40 g of TEOS was added. Then, the mixture was stirred for 24 h while the liquid temperature was maintained at 30°C, and then was cooled to a room temperature so as to form a second shell that encloses the silica capsule. Then, a water dispersion containing a silica capsule (A5) in which the model fragrance A was encapsulated in amorphous silica was obtained. The median diameter D50 of the silica capsule (A5) was 5.8 µm.

### Comparative Example 1

### (Step 1-1)

0.30 g of QUARTAMIN 60W (product name, manufactured by Kao corporation; CTAC, active component 30% by mass) was diluted with 84.70 g of ion-exchanged water to prepare an aqueous phase component. To this aqueous phase component, an oil phase component, which was prepared by mixing 10 g of the model fragrance A with 5 g of TEOS, was added. the mixed solution was emulsified by using the homomixer set at a rotation speed of 7,000 rpm and then further emulsified after the rotation speed of the homomixer was changed to 8500 rpm. Then, an emulsion was obtained. here, the median diameter D₅₀ of emulsified droplets was 1.0 µm.

Next, after the pH of the obtained emulsion was adjusted to 3.8 by using 0.12 g of 0.2 N hydrochloric acid, the emulsion was transferred to a separable flask equipped with a stirring blade and a cooler. Then, while the liquid temperature was maintained at 30°C, stirring was performed at 200 rpm for 24 h to obtain a water dispersion containing a silica capsule (1-C1) that has a core made of the model fragrance A and a first shell made of silica.

As a result of ICP (inductively coupled plasma) analysis of the obtained water dispersion, Si was detected, and thus it was confirmed that a silica source was present in the water dispersion.

### (Step 2-2')

To 15 g taken out from the total amount of 100.12 g of the water dispersion obtained in the step 1-1, 0.41 g of TEOS was added. Then, the mixture was stirred for 24 h while the liquid temperature was maintained at 30°C, and then was cooled to a room temperature so as to form a second shell that encloses the silica capsule. Then, a water dispersion containing a silica capsule (AC1) in which the model fragrance A was encapsulated in amorphous silica was obtained. The median diameter D₅₀ of the silica capsule (AC1) was 4.1 µm.

### Comparative Example 2

### (Step 1-1)

1.27 g of QUARTAMIN 60W (product name, manufactured by Kao corporation; CTAC, active component 30% by mass) was diluted with 74.73 g of ion-exchanged water to prepare an aqueous phase component. To this aqueous phase component, an oil phase component, which was prepared by mixing 20 g of the model fragrance A with 4 g of TEOS, was added. The mixed solution was emulsified by using the homomixer set at a rotation speed of 7,000 rpm, and then further emulsified after the rotation speed of the homomixer was changed to 8,500 rpm. Then, an emulsion was obtained. Here, the average particle size of emulsified droplets was 1.1 µm.

After the pH of the obtained emulsion was adjusted to 3.8 by using 0.09 g of 0.2 N hydrochloric acid, the emulsion was transferred to a separable flask equipped with a stirring blade and a cooler. Then, while the liquid temperature was maintained at 30°C, stirring was performed at 200 rpm for 24 h to obtain a water dispersion containing a silica capsule (1-C2) that has a core made of the model fragrance A and a first shell made of silica.

As a result of ICP (inductively coupled plasma) analysis of the obtained water dispersion, Si was detected, and thus it was confirmed that a silica source was present in the water dispersion.

### (Step 2-2")

To 13 g taken out from the total amount of 100.09 g of the water dispersion obtained in the step 1-1, 0.20 g of EMULGEN 108 (product name, manufactured by Kao corporation; polyoxyethylene laurylether (nonionic surfactant), active component 100% by mass) was added to obtain the water dispersion of silica capsules. Next, to 13 g of the water dispersion, 0.42 g of TEOS was added. Then, the mixture was stirred for 24 h while the liquid temperature was maintained at 30°C, and then was cooled to a room temperature so as to form a second shell that encloses the silica capsule. Then, a water dispersion containing a silica capsule (AC2) in which the model fragrance A was encapsulated in amorphous silica was obtained. The median diameter D₅₀ of the silica capsule (AC2), that is, the average particle size, was 7.0 µm.

### [Evaluation of microcapsule]

### [Evaluation of encapsulation rate of fragrance component]

80 mg of each of the water dispersions containing silica capsules (A1) to (A5) and (AC1) and (AC2), which are obtained in Examples 1 to 5 and Comparative Examples 1 and 2, was scooped up with a pipette and was diluted with 50 g of ion-exchanged water, and then passed through a membrane filter (manufactured by Millipore, product name "Omnipore", product number "JAWP04700") so that silica capsules were collected on the membrane filter.

Further, the silica capsules were washed with 10 mL of ion-exchanged water, and then 10 mL of hexane on the membrane filter. Then, the silica capsules were immersed in 10 mL of acetonitrile containing dodecane as an internal standard at a concentration of 10 µg/mL, and were subjected to irradiation with ultrasonic waves for 60 min by using an ultrasonic irradiation device (manufactured by Branson, model "5510") under conditions of output power of 180 W, and oscillation frequency of 42 kHz so as to elute the fragrance within the silica capsules. This solution passed through a membrane filter (manufactured by Toyo Roshi Kaisha, Ltd., product name "DISMIC", model "13JP020AN") again. Then, each fragrance component contained in this solution was measured by using gas chromatography, and was set as the amount α of the fragrance component encapsulated in the silica capsules. Next, the encapsulation rate of the fragrance component was calculated according to the following formula. The results of Examples 1 to 5 and Comparative Examples 1 and 2 are noted in Table 2 below.

Encapsulation rate of fragrance component (%)={(amount α of fragrance component encapsulated in silica capsules)/(amount β of fragrance component contained in 80 mg of water dispersion of silica capsules)}×100

The amount β of the fragrance component in the above formula was calculated from the composition of the used model fragrance, and the content in the water dispersion of the silica capsules.

**Table 2**

| | Step 1-1 | | | | | | Step 2-2 | | | Evaluation | | | | | | |
|---|---|---|---|---|---|---|---|---|---|---|---|---|---|---|---|---|
| | Aqueous phase component | | Oil phase component | | Emulsion | Initial pH of sol-gel reaction | Type of anionic surfactant | Addition amount (% by mass) of anionic surfactant *3 | Addition amount (% by mass) of silica source (TEOS) *4 | Type of silica capsule | Encapsulation rate of fragrance component contained in model fragrance A (% by mass) | | | | | |
| | Type of cationic surfactant | Addition amount (% by mass) of cationic surfactant *1 | Type of organic compound | Addition amount (% by mass) of silica source (TEOS) *2 | Median diameter D50 (um) of emulsified droplets | | | | | | Linalool | Rose oxide | Mentho ne | Citronel lol | Geraniol | Linalyl acetate |
| Example 1 | CTAC | 0.75 | Model fragrance A | 25 | 1.1 | 3.8 | EMAL 125A | 0.33 | 3.3 | A1 | 54 | 54 | 53 | 65 | 52 | 76 |
| Example 2 | CTAC | 1.88 | Model fragrance A | 25 | 1.0 | 3.8 | EMAL 125A | 0.31 | 3.0 | A2 | 68 | 66 | 67 | 77 | 70 | 85 |
| Example 3 | CTAC | 1.88 | Model fragrance A | 25 | 1.0 | 3.8 | LATEMU LE -108MB | 0.31 | 3.1 | A3 | 73 | 73 | 73 | 75 | 72 | 79 |
| Example 4 | CTAC | 1.88 | Model fragrance A | 25 | 1.0 | 3.8 | EMAL TD | 0.40 | 3.0 | A4 | 41 | 54 | 48 | 44 | 40 | 62 |
| Example 5 | CTAC | 1.88 | Model fragrance A | 25 | 1.0 | 3.8 | EMAL 20T | 0.40 | 3.0 | A5 | 40 | 42 | 42 | 41 | 36 | 53 |
| Comparative Example 1 | CTAC | 0.90 | Model fragrance A | 50 | 1.0 | 3.8 | - | 0 | (2.7) | AC1 | 23 | 23 | 22 | 13 | 6 | 33 |
| Comparative Example 2 | CTAC | 1.91 | Model fragrance A | 20 | 1.1 | 3.8 | EMULGE N 108 *5 | (1.54) | (3.2) | AC2 | 9 | 12 | 7 | 23 | 8 | 47 |

| | | | | | | | | | | | | | | | | |
|---|---|---|---|---|---|---|---|---|---|---|---|---|---|---|---|---|
| *1: The amount (% by mass) of the cationic surfactant used for producing the silica capsule (1) water dispersion, based on the amount of the organic compound in Step 1-1 *2: The amount (% by mass) of the silica source (TEOS) added in order to form the silica capsule (1), based on the amount of the organic compound in Step 1-1 *3: The addition amount (% by mass) of the anionic surfactant based on the silica capsule (1) water dispersion in Step 2-2 *4: The addition amount (% by mass) of the silica source (TEOS) in Step 2-2 based on the water dispersion to which the anionic surfactant is added *5: Nonionic surfactant | | | | | | | | | | | | | | | | |

From Table 2, it can be found that the silica capsules obtained in Examples have a higher encapsulation rate of the fragrance component than those in Comparative Examples. Further, it can be found that as compared to those in Comparative Examples, the silica capsules obtained in Examples retain a high encapsulation rate and make stable encapsulation even though they were subjected to suction filtration during the encapsulation rate evaluation.

### Industrial Applicability

According to the production method of the present invention, it is possible to obtain silica capsules in which an organic compound as an active component, such as a fragrance, is stably encapsulated at a high encapsulation rate, and thus the silica capsules are suitably used for various products with which an active component such as a fragrance is blended.

## Claims

1. A method of producing a microcapsule that comprises a shell comprising silica as a constituent component and a core comprising at least one organic compound inside the shell, the method comprising:
step 1-1: subjecting an emulsion obtained by emulsifying an aqueous phase component comprising a cationic surfactant, and an oil phase component comprising at least one organic compound and a silica source to a sol-gel reaction under an acidic condition to form a silica microcapsule (1) that has a core and a first shell whose constituent component is silica, and to obtain a water dispersion comprising the silica microcapsule (1), and
step 2-1: adding an anionic surfactant to the water dispersion obtained in the step 1-1, and then performing a sol-gel reaction in the presence of a silica source to form a microcapsule having a shell that encloses the silica microcapsule (1).

2. The method of producing a microcapsule according to claim 1, wherein the anionic surfactant in the step 2-1 is one or more selected from the group consisting of sulfate ester salts, sulfonates, alkenyl succinate, fatty acid salt having 8 or more and 22 or less carbon atoms, salts of aromatic sulfonic acid formalin condensate, acrylic acid homopolymers, acrylic acid copolymers, maleic acid homopolymers, and maleic acid copolymers.

3. The method of producing a microcapsule according to claim 1 or 2, wherein an amount of the anionic surfactant to be added in the step 2-1 is 0.1% by mass or more and 3% by mass or less based on the amount of the water dispersion comprising the silica microcapsule (1).

4. A method of producing a microcapsule that comprises a shell comprising silica as a constituent component and a core comprising at least one organic compound inside the shell, the method comprising:
Step 1-1: subjecting an emulsion obtained by emulsifying an aqueous phase component comprising a cationic surfactant, and an oil phase component comprising at least one organic compound and a silica source to a sol-gel reaction under an acidic condition to form a silica microcapsule (1) that has a core and a first shell whose constituent component is silica, and to obtain a water dispersion comprising the silica microcapsule (1), and
Step 2-2: adding an anionic surfactant and a silica source to the water dispersion obtained in the step 1-1, and then performing a sol-gel reaction of the silica source, to form a microcapsule having a second shell that encloses the silica microcapsule (1).

5. The method of producing a microcapsule according to claim 1 or 4, wherein an amount of the silica source to be added in order to form the silica microcapsule (1) in the step 1-1 is 10% by mass or more and 100% by mass or less based on the amount of the organic compound in the step 1-1.

6. The method of producing a microcapsule according to claim 1, 4 or 5, wherein the cationic surfactant in the step 1-1 is an alkyltrimethyl ammonium salt having an alkyl group having 10 or more and 22 or less carbon atoms.

7. The method of producing a microcapsule according to any one of claims 1 and 4 to 6, wherein the anionic surfactant in the step 2-1 and the step 2-2 is one or more selected from the group consisting of sulfate ester salts, sulfonates, alkenyl succinate, fatty acid salt having 8 or more and 22 or less carbon atoms, salts of aromatic sulfonic acid formalin condensate, acrylic acid homopolymers, acrylic acid copolymers, maleic acid homopolymers, and maleic acid copolymers.

8. The method of producing a microcapsule according to any one of claims 1 and 4 to 7, wherein an amount of the anionic surfactant to be added in the step 2-1 and the step 2-2 is 0.1% by mass or more and 3% by mass or less based on the amount of the water dispersion comprising the silica microcapsule (1).

9. The method of producing a microcapsule according to any one of claims 4 to 8, wherein an amount of the silica source to be added in the step 2-2 is 5% by mass or more and 100% by mass or less based on the amount of the organic compound in the step 1-1.

10. The method of producing a microcapsule according to any one of claims 1 and 4 to 9, wherein a median diameter D₅₀ of emulsified droplets of the emulsion in the step 1-1, as determined using a laser diffraction/scattering particle diameter distribution measuring device, is 0.1 µm or more and 10 µm or less.

11. The method of producing a microcapsule according to any one of claims 1, 4 to 10, wherein an initial pH in the sol-gel reaction in the step 1-1 is 3.0 or more and 4.5 or less.

12. The method of producing a microcapsule according to any one of claims 1 to 11, wherein the organic compound comprised in the core is one or more selected from the group consisting of fragrances, fragrance precursors, oil agents, antioxidants, antibacterial agents, fertilizers, surface modifiers, and solvents.

13. The method of producing a microcapsule according to any one of claims 1 to 12, wherein the silica source comprises tetraalkoxysilane in an amount of 80% by mass or more .

## Patentansprüche

1. Verfahren zur Herstellung einer Mikrokapsel, die eine Hülle, umfassend Siliciumoxid als Bestandteil, und einen Kern, umfassend mindestens eine organische Verbindung, innerhalb der Hülle umfasst, wobei das Verfahren umfasst:
Schritt 1-1: das Unterziehen einer Emulsion, erhalten durch Emulgieren einer wässrigen Phasenkomponente, umfassend ein kationisches Tensid, und einer Ölphasenkomponente, umfassend mindestens eine organische Verbindung und eine Siliciumoxidquelle, einer Sol-Gel-Reaktion unter sauren Bedingungen, um eine Siliciumoxid-Mikrokapsel (1) zu bilden, die einen Kern und eine erste Hülle, deren Bestandteil Siliciumoxid ist, aufweist, und um eine Wasserdispersion zu erhalten, die die Siliciumoxid-Mikrokapsel (1) umfasst, und
Schritt 2-1: das Hinzufügen eines anionischen Tensids zu der in Schritt 1-1 erhaltenen Wasserdispersion und das anschließende Durchführen einer Sol-Gel-Reaktion in Gegenwart einer Siliciumoxidquelle, um eine Mikrokapsel mit einer Hülle, die die Siliciumoxid-Mikrokapsel (1) umschließt, zu bilden.

2. Verfahren zur Herstellung einer Mikrokapsel gemäß Anspruch 1, wobei das anionische Tensid in Schritt 2-1 eines oder mehrere, ausgewählt aus der Gruppe, bestehend aus Sulfatestersalzen, Sulfonaten, Alkenylsuccinat, Fettsäuresalz mit 8 oder mehr und 22 oder weniger Kohlenstoffatomen, Salzen von aromatischem Sulfonsäure-Formalin-Kondensat, Acrylsäure-Homopolymeren, Acrylsäure-Copolymeren, Maleinsäure-Homopolymeren und Maleinsäure-Copolymeren, ist.

3. Verfahren zur Herstellung einer Mikrokapsel gemäß Anspruch 1 oder 2, wobei eine Menge des anionischen Tensids, die in Schritt 2-1 hinzugefügt wird, 0,1 Massen-% oder mehr und 3 Massen-% oder weniger, bezogen auf die Menge der Wasserdispersion, die die Siliciumoxid-Mikrokapsel (1) umfasst, beträgt.

4. Verfahren zur Herstellung einer Mikrokapsel, die eine Hülle, umfassend Siliciumoxid als Bestandteil, und einen Kern, umfassend mindestens eine organische Verbindung, innerhalb der Hülle umfasst, wobei das Verfahren umfasst:
Schritt 1-1: das Unterziehen einer Emulsion, erhalten durch Emulgieren einer wässrigen Phasenkomponente, umfassend ein kationisches Tensid, und einer Ölphasenkomponente, umfassend mindestens eine organische Verbindung und eine Siliciumoxidquelle, einer Sol-Gel-Reaktion unter sauren Bedingungen, um eine Siliciumoxid-Mikrokapsel (1) zu bilden, die einen Kern und eine erste Hülle, deren Bestandteil Siliciumoxid ist, aufweist, und um eine Wasserdispersion zu erhalten, die die Siliciumoxid-Mikrokapsel (1) umfasst, und
Schritt 2-2: das Hinzufügen eines anionischen Tensids und einer Siliciumoxidquelle zu der in Schritt 1-1 erhaltenen Wasserdispersion und das anschließende Durchführen einer Sol-Gel-Reaktion der Siliciumoxidquelle, um eine Mikrokapsel mit einer zweiten Hülle, die die Siliciumoxid-Mikrokapsel (1) umschließt, zu bilden.

5. Verfahren zur Herstellung einer Mikrokapsel gemäß Anspruch 1 oder 4, wobei eine Menge der Siliciumoxidquelle, die zur Bildung der Siliciumoxid-Mikrokapsel (1) in Schritt 1-1 hinzugefügt wird, 10 Massen-% oder mehr und 100 Massen-% oder weniger, bezogen auf die Menge der organischen Verbindung in Schritt 1-1, beträgt.

6. Verfahren zur Herstellung einer Mikrokapsel gemäß Anspruch 1, 4 oder 5, wobei das kationische Tensid in Schritt 1-1 ein Alkyltrimethylammoniumsalz mit einer Alkylgruppe mit 10 oder mehr und 22 oder weniger Kohlenstoffatomen ist.

7. Verfahren zur Herstellung einer Mikrokapsel gemäß einem der Ansprüche 1 und 4 bis 6, wobei das anionische Tensid in Schritt 2-1 und Schritt 2-2 eines oder mehrere, ausgewählt aus der Gruppe, bestehend aus Sulfatestersalzen, Sulfonaten, Alkenylsuccinat, Fettsäuresalz mit 8 oder mehr und 22 oder weniger Kohlenstoffatomen, Salzen von aromatischem Sulfonsäure-Formalin-Kondensat, Acrylsäure-Homopolymeren, Acrylsäure-Copolymeren, Maleinsäure-Homopolymeren und Maleinsäure-Copolymeren, ist.

8. Verfahren zur Herstellung einer Mikrokapsel gemäß einem der Ansprüche 1 und 4 bis 7, wobei eine Menge des anionischen Tensids, die in Schritt 2-1 und Schritt 2-2 hinzugefügt wird, 0,1 Massen-% oder mehr und 3 Massen-% oder weniger, bezogen auf die Menge der Wasserdispersion, die die Siliciumoxid-Mikrokapsel (1) umfasst, beträgt.

9. Verfahren zur Herstellung einer Mikrokapsel gemäß einem der Ansprüche 4 bis 8, wobei eine Menge der Siliciumoxidquelle, die in Schritt 2-2 hinzugefügt wird, 5 Massen-% oder mehr und 100 Massen-% oder weniger, bezogen auf die Menge der organischen Verbindung in Schritt 1-1, beträgt.

10. Verfahren zur Herstellung einer Mikrokapsel gemäß einem der Ansprüche 1 und 4 bis 9, wobei ein medianer Durchmesser D₅₀ der emulgierten Tröpfchen der Emulsion in Schritt 1-1, bestimmt unter Verwendung eines Laser-Beugungs-/Streuungs-Partikeldurchmessermessgeräts, 0,1 µm oder mehr und 10 µm oder weniger beträgt.

11. Verfahren zur Herstellung einer Mikrokapsel gemäß einem der Ansprüche 1, 4 bis 10, wobei ein anfänglicher pH-Wert in der Sol-Gel-Reaktion in Schritt 1-1 3,0 oder mehr und 4,5 oder weniger beträgt.

12. Verfahren zur Herstellung einer Mikrokapsel gemäß einem der Ansprüche 1 bis 11, wobei die im Kern enthaltene organische Verbindung eine oder mehrere, ausgewählt aus der Gruppe, bestehend aus Duftstoffen, Duftstoffvorläufern, Ölmitteln, Antioxidantien, antibakteriellen Mitteln, Düngemitteln, Oberflächenmodifizierungsmitteln und Lösungsmitteln, ist.

13. Verfahren zur Herstellung einer Mikrokapsel gemäß einem der Ansprüche 1 bis 12, wobei die Siliciumoxidquelle Tetraalkoxysilan in einer Menge von 80 Massen-% oder mehr umfasst.

## Revendications

1. Procédé de fabrication d'une microcapsule qui comprend une enveloppe comprenant de la silice comme composant constitutif et un noyau comprenant au moins un composé organique à l'intérieur de l'enveloppe, le procédé comprenant :
étape 1-1 : une soumission d'une émulsion obtenue en émulsionnant un composant en phase aqueuse comprenant un tensioactif cationique, et un composant en phase huileuse comprenant au moins un composé organique et une source de silice à une réaction sol-gel dans des conditions acides pour former une microcapsule de silice (1) qui présente un noyau et une première enveloppe dont le composant constitutif est la silice, et pour obtenir une dispersion aqueuse comprenant la microcapsule de silice (1), et
étape 2-1 : un ajout d'un tensioactif anionique à la dispersion aqueuse obtenue à l'étape 1-1, puis la mise en oeuvre d'une réaction sol-gel en présence d'une source de silice pour former une microcapsule présentant une enveloppe qui renferme la microcapsule de silice (1).

2. Procédé de fabrication d'une microcapsule selon la revendication 1, dans lequel le tensioactif anionique de l'étape 2-1 est un ou plusieurs composés sélectionnés parmi le groupe constitué par des sels d'esters sulfates, des sulfonates, du succinate d'alcényle, du sel d'acide gras présentant 8 ou plus et 22 ou moins atomes de carbone, des sels de condensat de formol d'acide sulfonique aromatique, des homopolymères d'acide acrylique, des copolymères d'acide acrylique, des homopolymères d'acide maléique, et des copolymères d'acide maléique.

3. Procédé de fabrication d'une microcapsule selon la revendication 1 ou la revendication 2, dans lequel une quantité du tensioactif anionique à ajouter à l'étape 2-1 est de 0,1 % en masse ou plus et de 3 % en masse ou moins sur la base de la quantité de la dispersion aqueuse comprenant la microcapsule de silice (1).

4. Procédé de fabrication d'une microcapsule qui comprend une enveloppe comprenant de la silice comme composant constitutif et un noyau comprenant au moins un composé organique à l'intérieur de l'enveloppe, le procédé comprenant :
étape 1-1 : une soumission d'une émulsion obtenue en émulsionnant un composant en phase aqueuse comprenant un tensioactif cationique, et un composant en phase huileuse comprenant au moins un composé organique et une source de silice à une réaction sol-gel dans des conditions acides pour former une microcapsule de silice (1) qui présente un noyau et une première enveloppe dont le composant constitutif est la silice, et pour obtenir une dispersion aqueuse comprenant la microcapsule de silice (1), et
étape 2-2 : un ajout d'un tensioactif anionique et d'une source de silice à la dispersion aqueuse obtenue à l'étape 1-1, puis une mise en oeuvre d'une réaction sol-gel de la source de silice, pour former une microcapsule présentant une seconde enveloppe qui renferme la microcapsule de silice (1).

5. Procédé de fabrication d'une microcapsule selon la revendication 1 ou la revendication 4, dans lequel une quantité de la source de silice à ajouter afin de former la microcapsule de silice (1) dans l'étape 1-1 est de 10 % en masse ou plus et de 100 % en masse ou moins sur la base de la quantité du composé organique dans l'étape 1-1.

6. Procédé de fabrication d'une microcapsule selon la revendication 1, la revendication 4 ou la revendication 5, dans lequel le tensioactif cationique dans l'étape 1-1 est un sel d'alkyltriméthylammonium présentant un groupe alkyle présentant 10 ou plus et 22 ou moins atomes de carbone.

7. Procédé de fabrication d'une microcapsule selon l'une quelconque des revendications 1 et 4 à 6, dans lequel le tensioactif anionique dans l'étape 2-1 et l'étape 2-2 est un ou plusieurs composés sélectionnés parmi le groupe constitué par des sels d'esters sulfates, des sulfonates, du succinate d'alcényle,
du sel d'acide gras présentant 8 ou plus et 22 ou moins atomes de carbone, des sels de condensat de formol d'acide sulfonique aromatique, des homopolymères d'acide acrylique, des copolymères d'acide acrylique, des homopolymères d'acide maléique, et des copolymères d'acide maléique.

8. Procédé de fabrication d'une microcapsule selon l'une quelconque des revendications 1 et 4 à 7, dans lequel une quantité du tensioactif anionique à ajouter dans l'étape 2-1 et l'étape 2-2 est de 0,1 % en masse ou plus et de 3 % en masse ou moins sur la base de la quantité de la dispersion aqueuse comprenant la microcapsule de silice (1).

9. Procédé de fabrication d'une microcapsule selon l'une quelconque des revendications 4 à 8, dans lequel une quantité de la source de silice à ajouter dans l'étape 2-2 est de 5 % en masse ou plus et de 100 % en masse ou moins sur la base de la quantité du composé organique dans l'étape 1-1.

10. Procédé de fabrication d'une microcapsule selon l'une quelconque des revendications 1 et 4 à 9, dans lequel un diamètre médian D₅₀ de gouttelettes émulsionnées de l'émulsion dans l'étape 1-1, tel que déterminé en utilisant un dispositif de mesure de distribution de diamètre de particule par diffraction/diffusion laser, est de 0,1 µm ou plus et de 10 µm ou moins.

11. Procédé de fabrication d'une microcapsule selon l'une quelconque des revendications 1, 4 à 10, dans lequel un pH initial dans la réaction sol-gel dans l'étape 1-1 est de 3,0 ou plus et de 4,5 ou moins.

12. Procédé de fabrication d'une microcapsule selon l'une quelconque des revendications 1 à 11, dans lequel le composé organique compris dans le noyau est un ou plusieurs composés sélectionnés parmi le groupe constitué par des parfums, des précurseurs de parfum, des agents huileux, des antioxydants, des agents antibactériens, des engrais, des modificateurs de surface, et des solvants.

13. Procédé de fabrication d'une microcapsule selon l'une quelconque des revendications 1 à 12, dans lequel la source de silice comprend du tétraalcoxysilane en une quantité de 80 % en masse ou plus.
